# EUROPEAN PATENT APPLICATION

(11) **EP 1 669 443 A1**
(43) Date of publication of application: **14.06.2006**
(21) Application number: 04773238.3
(22) Date of filing: 10.09.2004
(51) Int. Cl.: C12N 15/00, C07K 14/415, A01H 1/00, A01H 5/00

(54) **PLANT DWARFING GENE**

(30) Priority: 12.09.2003 JP 2003321497
(71) Applicant: RIKEN, Wako-shi, Saitama 351-0198 (JP); Nec Soft, LTD., Tokyo 136-8627 (JP)
(72) Inventor: MATSUI, Minami, Yokohama-shi, Kanagawa 2200054 (JP); NAKAZAWA, Miki, Yokohama-shi, Kanagawa 2310867 (JP); ICHIKAWA, Takanari, Yokosuka-shi, Kanagawa2390824 (JP); MUTO, Shu, c/o Nec Soft, Ltd., Tokyo 136-8627 (JP)
(74) Representative: Soames, Candida Jane
(86) International application number: PCT/JP2004/013594
(87) International publication number: WO 2005/026345

(57) **Abstract**

An object of the present invention is to explore, isolate, and identify a dwarfing gene by activation tagging. The present invention provides a gene encoding a protein of the following (a) or (b): (a) a protein consisting of the amino acid sequence represented by any of SEQ ID NOs: 2, 4, 6, and 8; or (b) a protein consisting of an amino acid sequence derived from the amino acid sequence represented by any of SEQ ID NOs: 2, 4, 6, and 8 with the deletion, substitution, or addition of one or several amino acids and having the activity of dwarfing a plant body.

## Description

### Technical Field

The present invention relates to a gene having the function of dwarfing a plant, a transformed plant with the gene introduced therein, and so on.

### Background Art

Mutants that link genes with phenotypes can be used for identifying gene function. There exist two types of mutants, loss-of-function mutants and gain-of-function mutants.

Loss-of-function mutations have been used for identifying gene function in many organisms. Many mutants were also characterized in *Arabidopsis thaliana* in the past few years, and most of them result from functionally recessive or loss-of-function mutations. The genomic sequence *of Arabidopsis thaliana* was determined, and it has been clear that most of its genes belong to families or have sequences closely related on the genome.

Activation tagging is a technique to analyze unknown gene function, which has been developed for *Arabidopsis thaliana,* and makes it possible to obtain gain-of-function mutants. T-DNA tagging vectors used in activation tagging have enhancers derived from cauliflower mosaic virus (CaMV) 35S promoter and was originally developed by Walden et al (Hayashi, H. et al., Science, 258, 1350-1353, 1992). T-DNA contains the CaMV 35S enhancers tandem arranged at a site proximal to the right border. The *Agrobacterium-mediated* introduction of this T-DNA into the plant genome activates and overexpresses genes near the insertion site by the action of the enhancers, with the result that a change occurs in the phenotype of the plant body. For example, a gene involved in hormone signaling (Kakimoto, T. et al., Science, 274, 982-985, 1996) and a gene involved in early flowering phenotypes (Kardailsky, I. et al., Science, 286, 1962-1965, 1999) have previously been reported to be isolated from *Arabidopsis thaliana* by utilizing this activation tagging.

Activation tagging has such advantages that: (i) all activation-tagged mutants are dominant and therefore permit the screening of phenotypes in the T₁ generation; (ii) mutations in redundantly acting genes can be expected to produce phenotypes; and (iii) there is a high possibility of linking activated genes with dominant mutant phenotypes.

Following the development of transformants *of Arabidopsis thaliana* by use of vacuum infiltration or flower dipping technique, this T-DNA was introduced into plant bodies to create mutant lines or activation-tagged lines. The activation of genes by the CaMV 35S enhancers brings about dominant phenotypes. Several dominant mutants have previously been isolated from activation-tagged lines, and some of them have been shown to be caused by mutations in genes belonging to families. The present inventors have already isolated a dominant mutant dfl1-D involved in auxin response and photomorphogenesis as well as the corresponding gene to this mutation that encodes a soybean GH3 homologue, from the screening of activation-tagged lines of *Arabidopsis thaliana* (Nakazawa, M. et al., Plant J. 25, 213-221, 2001; and JP Patent Publication (Kokai) No. 2002-10786).

An object of the present invention is to explore, isolate, and identify a dwarfing gene by activation tagging.

### Disclosure of the Invention

The present inventors screened morphological mutants from the T₁ generations of approximately 50,000 activation-tagged lines of *Arabidopsis thaliana* and successively isolated genes associated with plant dwarfing, thereby completing the present invention.

Namely, the present invention encompasses the following inventions.
(1) a gene encoding a protein of the following (a) or (b):
   (a) a protein consisting of the amino acid sequence represented by any of SEQ ID NOs: 2, 4, 6, and 8; or
   (b) a protein consisting of an amino acid sequence derived from the amino acid sequence represented by any of SEQ ID NOs: 2, 4, 6, and 8 with the deletion, substitution, or addition of one or several amino acids and having the activity of dwarfing a plant body.
(2) a gene comprising DNA of the following (c) or (d):
   (c) DNA consisting of the nucleotide sequence represented by any of SEQ ID NOs: 1, 3, 5, and 7; or
   (d) DNA hybridizing under stringent conditions to DNA consisting of a nucleotide sequence complementary to DNA consisting of the nucleotide sequence represented by any of SEQ ID NOs: 1, 3, 5, and 7 and encoding a protein having the activity of dwarfing a plant body.
(3) a protein of the following (a) or (b):
   (a) a protein consisting of the amino acid sequence represented by any of SEQ ID NOs: 2, 4, 6, and 8; or
   (b) a protein consisting of an amino acid sequence derived from the amino acid sequence represented by any of SEQ ID NOs: 2, 4, 6, and 8 with the deletion, substitution, or addition of one or several amino acids and having the activity of dwarfing a plant body.
(4) a recombinant vector comprising a gene according to (1) or (2).
(5) a transformed plant with a gene according to (1) or (2) or a recombinant vector according to (4) introduced therein.
(6) the transformed plant according to (5), wherein the plant is a plant body, plant organ, plant tissue, or cultured plant cell.
(7) the transformed plant according to (5) or (6), wherein the plant belongs to any family selected from the group consisting of the families *Brassicaceae, Solanaceae, Poaceae,* and *Leguminosae*.
(8) a method for dwarfing a plant body by inducing the overexpression of a gene according to (1) or (2) in the plant body.

### Brief Description of the Drawings

Fig. 1 shows a map of a T-DNA insertion site in the genome of a tagged line Z029218.
Fig. 2 shows a map of a T-DNA insertion site in the genome of a tagged line Z043547.
Fig. 3 shows a map of a T-DNA insertion site in the genome of a tagged line Z093001.
Fig. 4 shows a map of a T-DNA insertion site in the genome of a tagged line Z029732/Z068035.
Fig. 5 shows a photograph of an At4g31910 transformant (6-week old).
Fig. 6 is a photograph showing the comparison (6-week old) between an At4g31910 transformant (right) and a wild strain (left).
Fig. 7 shows a photograph of an At1g04910 transformant (6-week old).
Fig. 8 shows a photograph of an At1g04910 transformant (6-week old).
Fig. 9 shows a photograph of an At4g3 5 700 transformant (6-week old).
Fig. 10 shows a photograph of an At4g35700 transformant (6-week old).
Fig. 11 shows a photograph of an At1g49770 transformant (6-week old).
Fig. 12 shows a photograph of an At1g49770 transformant (6-week old).
Fig. 13 shows a photograph of separately dried wild-type and At1g49770 transformant seeds (OX-1 to -8 denote the designations of At1g49770 transformant lines; and the left and the right in each panel show the wild-type seeds (controls) and the At1g49770 transformant seeds, respectively).
Fig. 14 shows a result of tannin detection by the DMACA staining of At1g49770 transformant seeds (OX-1 to -8 denote the Atlg49770 transformant seeds; and Col denotes wild-type seeds (controls)).

Hereinafter, the present invention will be described in detail. The present application claims the priority of Japanese Patent Application No. 2003-321497 filed on Sep. 12, 2003 and encompasses contents described in the specification and/or drawings of the patent application.

### 1. Isolation of gene of the present invention

### (1) Activation tagging technique

A gene of the present invention can be obtained by producing transcriptionally activated mutants of plant genes by activation tagging technique, and cloning the causative genes.

Specifically, it can be achieved by the following procedures:
(i) an activation-tagging T-DNA vector is randomly inserted through *Agrobacterium* into the genome *ofArabidopsis thaliana* to produce activation-tagged lines;
(ii) T₁ plants are grown from seeds harvested from the tagged lines, and their phenotypic traits are recorded on the basis of predetermined inspection items for phenotypic traits, simultaneously with digital imaging thereof;
(iii) DNA fragments containing the T-DNA are collected by plasmid rescue from the genomes of the mutant strains whose phenotypic traits clearly differ in the T₁ generation from those of wild types, and are then sequenced;
(iv) the DNA fragments are introduced into wild-type *Arabidopsis thaliana* to examine whether the mutant phenotypic trait can be reproduced; and
(v) the corresponding cDNA is cloned.

In the present specification, the "T₁ generation" means a plant generation obtained from seeds of plants of the "T₀ generation", a transformed plant generation. The "T₁ generation" is the first population from the transformed plants and can be selected using selective agents (e.g., antibiotics and herbicides) compatible with the resistant genes of the transgenic plants. Alternatively, the "T₂ generation" means a plant generation obtained by the self-pollination of flowers of the "T₁ generation" plants preselected as transgenic plants.

pPCVICEn4HPT developed by Walden et al (Hayashi, H. et al, Science, 258, 1350-1353, 1992) can be used as the activation-tagging T-DNA vector. This vector is a binary vector that tandem possesses 4 enhancers (-90 to -440) in CaMV 35S promoter near the RB. *Arabidopsis thaliana* is transformed with *Agrobacterium* GV3101 (pMP90RK) bearing this pPCVICEn4HPT. The transformation can be performed by flower dipping technique that involves immersing and coculturing the above ground part *of Arabidopsis thaliana* in a suspension of *Agrobacterium.*

Examples of phenotypic traits observed in the T₁ plants include plant body height, rosette color, the number of leaves before bolting, leaf width, petiole length, flower shape, the number of floral organs, flowering time, and the fertility and blanching of shoots.

### (2) Plasmid rescue technique and functional assay of resulting DNA fragments

If interesting mutants are obtained, genes that cause the mutations by transcriptional activation are cloned. Plasmid rescue technique is preferred as a method for the cloning. Specifically, DNAs of the mutants are purified and treated with a variety of restriction enzymes. Band size is confirmed by southern blot to find restriction enzymes that offer approximately 10- to 20-kb fragments containing the inserted T-DNA. Next, the DNAs are treated with those restriction enzymes and subsequently with phenol/chloroform. The resulting DNAs are precipitated with ethanol and then self-ligated with ligase. These DNAs are introduced by electroporation into competent cells *(Escherichia coli* DH10B). After the selection of resistant strains on a medium containing ampicillin, plasmids are selected by a routine method. A genomic DNA boundary sequence adjacent to the inserted T-DNA contained in the obtained plasmid is determined, and the position of T-DNA insertion on the genome is determined. Based on the position, genes having translation initiation sites within 6 kb from the enhancer sequences are searched using *Arabidopsis thaliana* genome database (http://www.mips.biochem.mpg.de). These genes are used as candidate genes to design primers specific to the genes or recombinant vectors introduced into the plants. cDNAs are amplified from an *Arabidopsis thaliana* cDNA library, and the resulting amplified fragments are cloned. These cDNA fragments are introduced through *Agrobacterium* into plants to examine whether the mutant phenotype is reproduced.

Although cDNA sequencing can be performed by an approach known in the art such as Maxam-Gilbert chemical modification method or dideoxynucleotide chain termination method using M13 phage, cDNA is typically sequenced using an automatic sequencer (e.g., ABI 373 sequencer and 310 DNA sequencer; both manufactured by Applied Biosystems). The obtained nucleotide sequences are analyzed with DNA analysis software such as DNASIS (Hitachi Software Engineering), and coding regions for encoded proteins can be found in the resulting DNA strands.

The gene of the present invention isolated and identified by the approach described above is At4g31910 (Z029218), At1g04910 (Z043547), At4g35700 (Z093001), and At1g49770 (Z029732, Z068035) (tagged-line designations are given in the parentheses). The nucleotide sequences of At4g31910, At1g04910, At4g35700, and At1g49770 are shown in SEQ ID NOs: 1, 3, 5, and 7, respectively. Alternatively, amino acid sequences encoded by At4g31910, At1g04910, At4g35700, and At1g49770 are shown in SEQ ID NOs: 2, 4, 6, and 8, respectively.

As long as a protein consisting of each amino acid sequence represented by any of SEQ ID NOs: 2, 4, 6, and 8 can dwarf a plant body, the amino acid sequence may have the mutations such as deletion, substitution, and addition of plural amino acids, preferably one or several amino acids.

For example, 1 to 10 amino acids, preferably 1 to 5 amino acids, may be deleted in the amino acid sequence represented by any of SEQ ID NOs: 2, 4, 6, and 8, may be added to the amino acid sequence represented by any of SEQ II7 NOs: 2, 4, 6, and 8, or may be substituted in the amino acid sequence represented by any of SEQ ID NOs: 2, 4, 6, and 8 by other amino acids.

The scope of the present invention also encompasses a gene that encodes a protein having 70% or more homology to the amino acid sequence represented by any of SEQ ID NOs: 2, 4, 6, and 8 and having the activity of dwarfing a plant body. The 70% or more homology refers to preferably 80% or more homology, more preferably 90% or more homology, most preferably 95% or more homology.

The deletion, addition, and substitution of the amino acids can be performed by modifying the gene encoding the protein by an approach known in the art. The mutations can be introduced into the gene by an approach known in the art such as Kunkel or Gapped duplex method or an equivalent method thereof. For example, a mutation-introducing kit utilizing site-specific mutagenesis (e.g., Mutant-K (manufactured by TAKARA) and Mutant-G (manufactured by TAKARA)) is used, or alternatively LA PCR in vitro Mutagenesis Series Kit available from TAKARA is used, to introduce the mutations.

In this context, the "activity of dwarfing a plant body" in the present invention means the activity of decreasing the height of a plant body (e.g., *Arabidopsis thaliana)* with the expression of the gene of the present invention to 2/3 to 1/10, preferably 1/3 to 1/10 those of wild types. The phrase "having the activity of dwarfing a plant body" means that the activity described above is substantially equal to the activity of the protein having the amino acid sequence represented by any of SEQ ID NOs: 2, 4, 6, and 8.

The gene of the present invention also encompasses DNA hybridizing under stringent conditions to DNA consisting of a nucleotide sequence complementary to DNA consisting of the nucleotide sequence represented by any of SEQ ID NOs: 1, 3, 5, and 7 and encoding a protein having the activity of dwarfing a plant body. In this context, the stringent conditions refer to conditions that provide for the formation of so-called specific hybrids but no nonspecific-hybrid. Examples of the stringent conditions include conditions under which the complementary strand of a nucleic acid having high homology, that is, DNA consisting of a nucleotide sequence having 90% or more homology, preferably 95% or more homology, to the nucleotide sequence represented by any of SEQ ID NOs: 1, 3, 5, and 7, is hybridized, while the complementary strand of a nucleic acid having lower homology is not hybridized. To be more specific, the stringent conditions refer to conditions at a sodium concentration of 15 to 300 mM, preferably 15 to 75 mM, and at a temperature of 50 to 60°C, preferably 55 to 60°C.

Once the nucleotide sequence of the gene of the present invention is definitely determined, the gene of the present invention can be obtained by chemical synthesis, by PCR using cloned cDNA as a template, or by the hybridization of a DNA fragment having the nucleotide sequence as a probe. In addition, modified DNA that encodes the gene can be synthesized by site-specific induction or the like.

### 2. Production of recombinant vector and transformed plant

### (1) Production of recombinant vector

A recombinant vector of the present invention can be produced by inserting the gene of the present invention into an appropriate vector. pBI-, pUC-, and pTRA-type vectors are preferably used as vectors for introducing and expressing the gene of the present invention into a plant cell. The pBI- and pTRA-type vectors allow the introduction of genes of interest into plants through *Agrobacterium.* pBI-type binary or intermediate vectors are preferably used, and examples thereof include pBI121, pBI101, pBI101.2, and pBI101.3. The pUC-type vectors allow the direct introduction of genes into plants, and examples thereof include pUC 18, pUC19, and pUC9. Alternatively, plant virus vectors such as cauliflower mosaic virus (CaMV), bean golden mosaic virus (BGMV), tobacco mosaic virus (TMV) can also be employed.

For inserting the gene of the present invention into the vector, for example, a method is adopted in which purified DNA is initially cleaved with an appropriate restriction enzyme and then inserted into the restriction enzyme site or multicloning site of appropriate vector DNA, which is in turn ligated with the vector.

The gene of the present invention should be incorporated into the vector so that its gene function is exhibited. Therefore, the vector can be ligated, if desired, with an enhancer, splicing signal, poly(A)-addition signal, selective marker, 5'-UTR sequence, and so on, in addition to a promoter and the gene of the present invention. Examples of the selective marker include dihydrofolate reductase gene, ampicillin-resistant gene, neomycin-resistant gene, hygromycin-resistant gene, and bialaphos-resistant gene.

The "promoter" does not have to be derived from plants, as long as it is DNA that can function in plant cells and induce gene expression in particular plant tissues or in particular plant developmental stages. Concrete examples of the promoter include cauliflower mosaic virus (CaMV) 35S promoter, nopaline synthase gene promoter (Pnos), maize-derived ubiquitin promoter, rice-derived actin promoter, and tobacco-derived PR protein promoter.

A "terminator" may be a sequence that can terminate the transcription of genes transcribed by the aforementioned promoter. Concrete examples of the terminator include nopaline synthase gene terminator (Tnos) and cauliflower mosaic virus poly(A) terminator.

The "enhancer" is used for increasing the expression efficiency of genes of interest. For example, an enhancer region containing an upstream sequence in the CaMV 35S promoter is preferable.

### (2) Production of transformed plant

A transformed plant of the present invention can be obtained by introducing the gene of the present invention or the recombinant vector containing it into a host so as to allow the expression of the gene of interest. In this contest, the host is not particularly limited as long as it can express the gene of the present invention. However, a plant is preferable as the host. When the host is a plant, the transformed plant (transgenic plant) can be obtained in the way described below.

The plant to be transformed in the present invention means any of an entire plant body, a plant organ (e.g., leaves, petals, stems, roots, and seeds), a plant tissue (e.g., epidermis, phloem, parenchyma, xylem, and vascular bundle), and a cultured plant cell.

Examples of the plant used in transformation include, but not limited to, plants belonging to the families *Brassicaceae, Poaceae, Solanaceae,* and *Leguminosae* (see the list described below).
Family *Brassicaceae: Arabidopsis thaliana*
Family *Solanaceae*: *Nicotiana tabacum*
Family *Poacea: Zea mays and Oryza sativa*
Family *Leguminosae: Glycine max*

Examples of a method for introducing the gene or recombinant vector of the present invention into the plant include Agrobacterium, PEG-calcium phosphate, electroporation, liposome, particle gun, and microinjection techniques. For example, when the Agrobacterium technique is used, this technique utilizes protoplasts in some cases and tissue sections in other cases. When a protoplast is used, the protoplast is cultured together with the Agrobacterium having a Ti plasmid, or it is fused with a spheroplasted Agrobacterium (the spheroplast method). When a tissue section is used, Agrobacterium is allowed to infect an aseptically cultivated leaf section (a leaf disc) of target plant (the leaf disc method) or a callus (undifferentiated cultured cell).

Confirmation as to whether or not the gene is incorporated into the plant can be performed by PCR, southern hybridization, northern hybridization, or the like. For example, DNA is prepared from the transformed plant and subjected to PCR using designed DNA-specific primers. After PCR, the resulting amplification product is subjected to agarose gel electrophoresis, polyacrylamide gel electrophoresis, or capillary electrophoresis, or the like, and then stained with ethidium bromide, SYBR Green solution, or the like. The amplification product can be detected as a single band to thereby confirm that the transformation is successful. Alternatively, the amplification product can also be detected by performing PCR using primers labeled in advance with fluorochrome or the like. Furthermore, a method may be used in which the amplification product is bound to a solid phase such as microplates and confirmed by, for example, fluorescent or enzyme reaction.

Tumor tissues, shoots, hairy roots, and so on, obtained by transformation may be used directly in cell culture, tissue culture, or organ culture, or can be regenerated into plant bodies using a conventionally known plant tissue culture method by, for example, the administration of plant hormone (e.g., auxin, cytokinin, gibberellin, abscisic acid, ethylene, and brassinolide) with an appropriate concentration.

Although the transformed plants thus obtained are typically "dwarfed", they respectively have phenotypic traits described below.
At4g31910: small rosettes, round leaves, epinastic leaves, short petiole, short internodal spacings, the second inflorescence having a wide angle relative to the first inflorescence
At1g04910: small leaves, epinastic leaves, abnormal internodal spacings
At4g35700: small rosettes, round leaves, short internodal spacings
At1g49770: decreased apical dominance, short internodal spacings, decreased tannin contents

In the present specification, the term "phenotype" is used synonymously with the term "phenotypic trait". These terms mean the easily observable or measurable morphological characteristics of plants.

The phenotypic trait of a short height has such advantages that: plants having this phenotypic trait are resistant to wind damage such as typhoons and are less likely to fall down even by increased grains; for example, in the case of rice, the density of seedling planting per unit area can be rendered larger because the number of rows for seedlings to be planted can be increased; and when it is applied to fruit trees (e.g., banana and mango) and palm trees (e.g., date and coconut) that extend to a few meters in height, labors for harvesting fruits are alleviated, and yields per unit resource (e.g., water and fertilizers) are increased.

The phenotypic trait of unconventional leave or flower shape or size has such advantages as enhanced commercial value in cut flowers, foliage plants, and bonsai plants, and attractiveness to purchasers.

For example, At4g31910 is dwarfed and however, has a feature of the absence of decreases in the number of flowers and yields. It has advantages from both standpoints: horticulturally, the enhanced clustering of flowers, and agriculturally, dwarfing and the maintenance of yields.

Moreover, At1g49770 has a feature of decreased tannin contents. However, this should be noted in that there has been no report on means that can negatively control the expression level of tannin. For example, this feature allows the control of flower color, reduction in astringent taste, the inhibition and suppression of iron absorption, and so on.

### 3. Production of protein of the present invention

A protein of the present invention can be obtained by ligating (inserting) the gene of the present invention isolated in the paragraph 1 into a recombinant vector such as plasmid DNA and phage DNA capable of replication in hosts, and then introducing the vector into a host such as *Escherichia coli* other than plant hosts to give a transformant, which is in turn cultured, followed by collection from the resulting cultured product. In this contest, the "cultured product" means any of a culture supernatant, a cultured cell, a cultured microorganism, or a disrupted product of the cultured cell or microorganism.

Examples of the plasmid DNA include *Escherichia* coli-derived plasmids (e.g., pBR322, pBR325, pUC 118, pUC119, pUC 18, pUC19, and pBluescript), *Bacillus* subtilis-derived plasmids (e.g., pUB110 and pTP5), and yeast-derived plasmids (e.g., YEp13 and YCp50). Examples of the phage DNA include λ phages (e.g., Charon4A, Charon21A, EMBL3, EMBL4, λgt10, λgt11, and λZAP). In addition, animal viruses such as retroviruses or vaccinia viruses as well as insect virus vectors such as baculoviruses can also be employed.

For example, bacteria belonging to the genus *Escherichia* such as *Escherichia coli,* bacteria belonging to the genus *Bacillus* such as *Bacillus subtilis,* bacteria belonging to the genus *Pseudomonas* such as *Pseudomonas putida,* bacteria belonging to the genus *Rhizobium* such as *Rhizobizim meliloti,* yeasts such as *Saccharomyces cerevisiae* and *Schizosaccharomyces pombe,* animal cells such as COS and CHO cells, or insect cells such as Sf9 can be used as the host other than plant hosts.

When a bacterium such as *Escherichia coli* or a yeast is used as the host, it is preferred that the recombinant vector described above should be capable of autonomous replication in the bacterium and should be composed of a promoter, a ribosome-binding sequence, the gene of the present invention, and a transcription termination sequence. The recombinant vector may also contain a gene that controls the promoter.

Examples of the *Escherichia coli* include, but not limited to, *Escherichia coli* DH5α and HB101. Examples of the *Bacillus subtilis* include, but not limited to, *Bacillus subtilis.* In this case, the promoter is not particularly limited as long as it allows gene expression in the host such as *Escherichia coli.* For example, an *Escherichia coli-* or phage-derived promoter such as trp promoter, lac promoter, P_{L} promoter, and P_{R} promoter can be used. A method for introducing the recombinant vector into the bacterium is not particularly limited as long as it is a method that provides for the introduction of DNA into a bacterium. Examples thereof include a method that uses calcium ions (Cohen, S.N. et al., Proc. Natl. Acad. Sci., USA, 69: 2110 (1972)) as well as electroporation.

When a yeast is used as the host, for example, *Saccharomyces cerevisiae* or *Pichia pastoris* is used. In this case, the promoter is not particularly limited as long as it allows gene expression in the yeast. For example, gal1 promoter, gal10 promoter, heat shock protein promoter, MFα1 promoter, PHO5 promoter, PGK promoter, GAP promoter, ADH promoter, and AOX1 promoter can be used. A method for introducing the recombinant vector into the yeast is not particularly limited as long as it is a method that provides for the introduction of DNA into a yeast. Examples thereof include electroporation, spheroplast, and lithium acetate methods.

When an animal cell is used as the host, for example, a monkey cell COS-7 or Vero, Chinese hamster ovary (CHO) cell, or murine L cell is used. In this case, SRα promoter, SV40 promoter, LTR promoter, CMV promoter, or the like is used as the promoter, or otherwise, human cytomegalovirus early gene promoter or the like may also be employed as the promoter. Examples of a method for introducing the recombinant vector into the animal cell include electroporation, calcium phosphate, and lipofection methods.

When an insect cell is used as the host, Sf9 cell or the like is used. Examples of a method for introducing the recombinant vector into the insect cell include calcium phosphate, lipofection, and electroporation methods.

Procedures for culturing the transformant described above are performed according to a routine method used in host culture.

A medium used for culturing the transformant obtained from a microorganism such as *Escherichia coli* or a yeast used as the host may be any of natural and synthetic media as long as it contains carbon sources, nitrogen sources, inorganic salts, and so on capable of being assimilated by the microorganism and allows the efficient culture of the transformant. Examples of the carbon sources include: carbohydrates such as glucose, fructose, sucrose, and starch; organic acids such as acetic acid and propionic acid; and alcohols such as ethanol and propanol. Examples of the nitrogen sources include: ammonia; ammonium salts of inorganic or organic acids such as ammonium chloride, ammonium sulfate, ammonium acetate, and ammonium phosphate; other nitrogen-containing compounds; peptone; meat extracts; and corn steep liquor. Examples of inorganic substances include dipotassium hydrogen phosphate, potassium dihydrogen phosphate, magnesium phosphate, magnesium sulfate, sodium chloride, ferrous sulfate, manganese sulfate, copper sulfate, and calcium carbonate. The transformant is typically cultured at 37°C under aerobic conditions such as shaking culture or aeration agitation culture. pH adjustment is performed using an inorganic or organic acid, an alkali solution, or the like. During the culture, an antibiotic such as ampicillin or tetracycline may optionally be added to the medium.

When a microorganism transformed with an expression vector using an inducible promoter as the promoter is cultured, an inducer may optionally be added to the medium. For example, when a microorganism transformed with an expression vector having a promoter inducible by isopropyl-β-D-thiogalactopyranoside (IPTG) is cultured, IPTG or the like can be added to the medium. Alternatively, when a microorganism transformed with an expression vector using trp promoter inducible by indoleacetic acid (IAA) is cultured, IAA or the like can be added to the medium.

Examples of a medium used for culturing a transformant obtained from an animal cell used as the host include RPMI 1640 medium and DMEM medium generally used, or these media supplemented with fetal bovine serum. The transformant is typically cultured at 37°C for 1 to 30 days in the presence of 5% CO₂. During the culture, an antibiotic such as kanamycin or penicillin may optionally be added to the medium.

If the protein of the present invention is produced in the microorganism or cell after the culture, the protein of interest is collected by disrupting the microorganism or cell by ultrasonication, repetitive freeze-thaw, homogenizer treatment, or the like. Alternatively, if the protein of the present invention is produced outside the microorganism or cell, the culture solution is directly used or is subjected to centrifugation or the like to remove the microorganism or cell. Then, the protein of the present invention can be isolated and purified from the cultured product by using, alone or in an appropriate combination, general biochemical methods used in protein isolation and purification, for example, ammonium sulfate precipitation, gel chromatography, ion exchange chromatography, and affinity chromatography.

### 4. Method for dwarfing plant body

The present invention also provides a method for dwarfing a plant body by inducing the overexpression of the gene of the present invention in the plant body.

The method for dwarfing a plant according to the present invention is not particularly limited to methods using transformation and also encompasses, for example, a method that comprises administrating a substance or imparting environmental stress, to a plant in a developmental stage to induce the overexpression of the gene of the present invention, thereby dwarfing the plant.

### Best Mode for Carrying Out the Invention

Hereinafter, the present invention will be described more fully with reference to Examples. However, the present invention is not intended to be limited to these Examples. (Example 1) Production and gene analysis of mutants

### (1) Activation-tagged lines and plant growth conditions

An *Arabidopsis thaliana* wild type (Col-0) was grown at 22°C in long-day conditions (16-hour light and 8-hour dark conditions) under white fluorescent tubes (FL40SW; manufactured by Sanyo).

Plants were transformed by floral dipping technique with *Agrobacterium tumefaciens* GV3101 strains (pMP90RK) containing the activation-tagging T-DNA vector, pPCVICEn4HPT, provided by Dr. R. Walden (Clough, S.J. and Bent, A.F., Plant J., 16, 735-743, 1998; and Hayashi, H., et al., Science, 258, 1350-1353, 1992). Hygromycin-resistant T₁ seedlings were selected on a basic agar medium for 7 days containing 1 mM KNO₃, 0.8% agar, and 50 mg/l hygromycin and transferred into soil (Nakazawa, M. and Matsui, M., Biotechniques, 34, 28-30, 2003).

During the growth of T₁ plant, 3 strains of visible phenotype mutants were picked up (tagged lines Z029218, Z043547, and Z093001), from which some rosette leaves were harvested as a material for plasmid rescue.

### (2) Plasmid rescue and identification of T-DNA insertion site in the genome of Arabidopsis thaliana

About 200 mg of the rosette leaves were collected into screw-capped plastic tubes. Five ceramic particles (CERAMICS YTZ ball, D: 3 mm; manufactured by Nikkato) and 300 µl of lysis buffer were added to the plant material, which was then homogenized using Shake Master (manufactured by BioMedical Science). Genomic DNAs were extracted using Wizard Magnetic 96 DNA Plant System (manufactured by Promega). A workstation system (Tecan genesis workstation 150) was used for conducting the extraction protocol described in the extraction kit. The genomic DNAs were digested with BamHI in a volume of 100 µl overnight. The digested DNAs were precipitated with ethanol and dissolved in 8 µl of distilled water. The DNAs were self-ligated with T4 ligase (400 units/µl; manufactured by BioLabs) in a volume of 10 µl at room temperature overnight. *Escherichia coli* DH10B electro-competent cells (20 µl; manufactured by Invitrogen) were transformed by electroporation with 2 µl of the ligation solution and spread on LB medium containing ampicillin. After incubation at 38°C overnight, 8 colonies per line were selected and subcultured in 5 ml of LB liquid medium containing ampicillin. Plasmid DNAs were prepared from the resulting liquid medium and digested with HindIII to distinguish plasmids that did not contain genomic fragments.

The genomic fragments in the plasmids were sequenced using LB2 primer (5'-TGACCATCATACCCATTGCTGATCC-3'). The obtained sequencing results were analyzed using the BLASTN program against the NCBI non-redundant database to identify the position of T-DNA insertion sites in the genome *of Arabidopsis thaliana*.

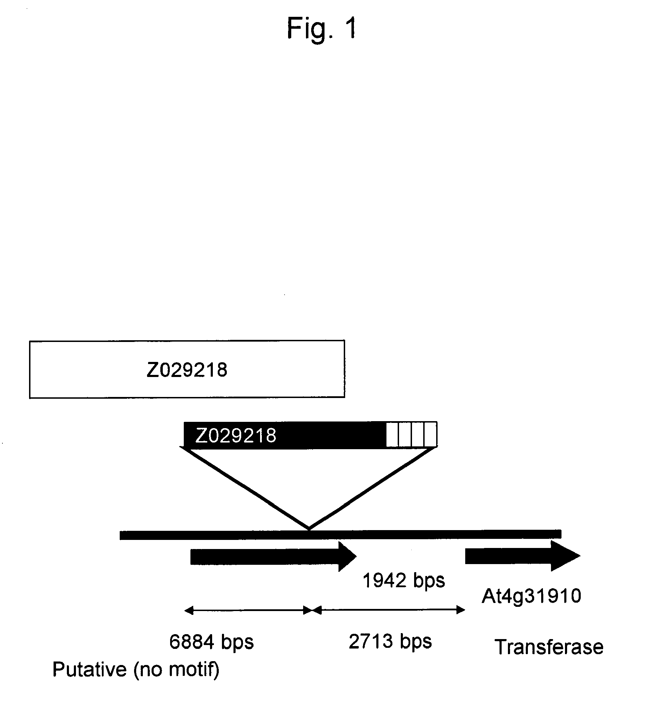

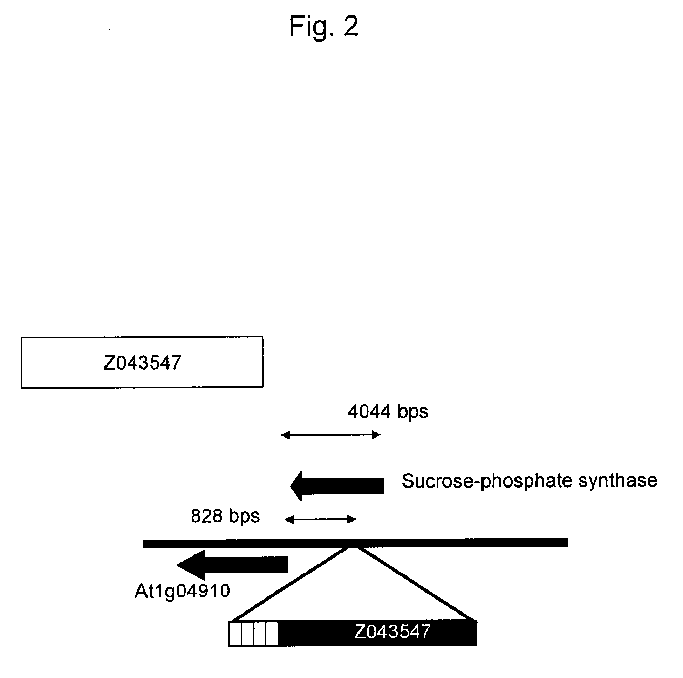

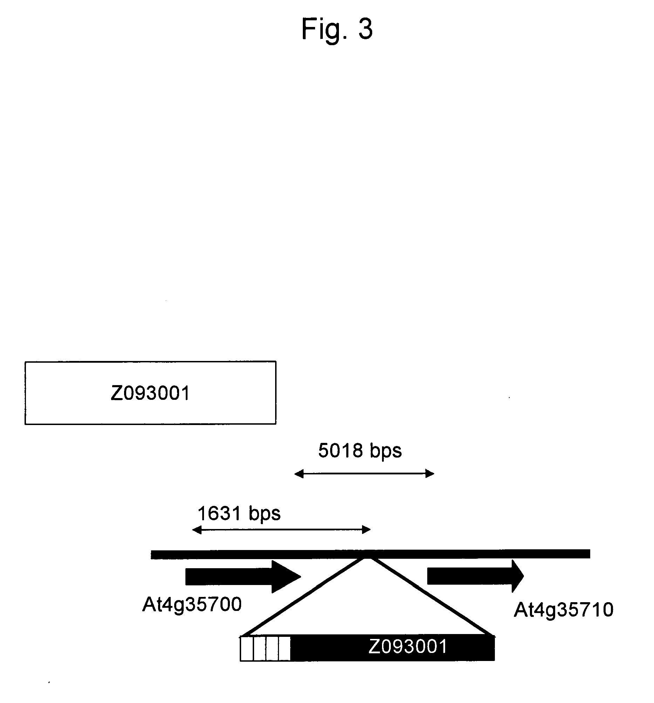

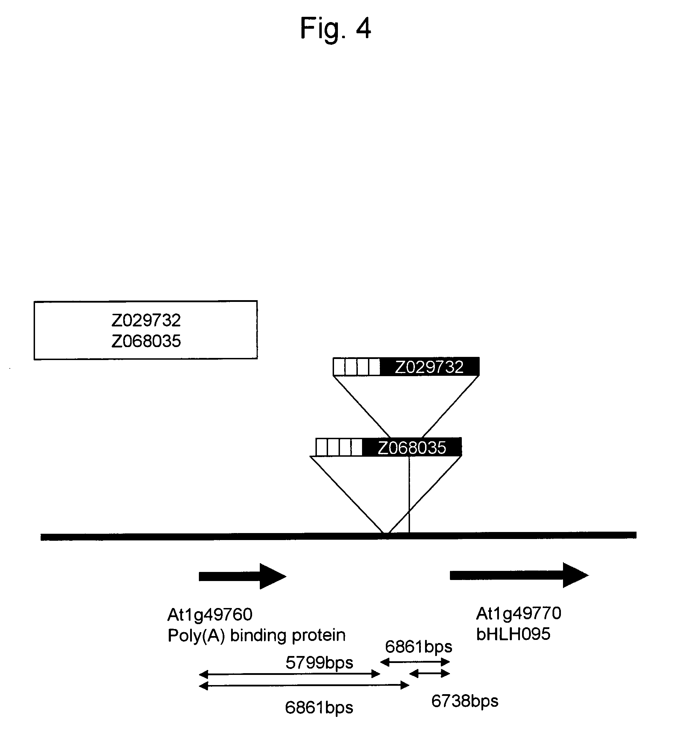

The insertion sites on the genomes of mutants (tagged lines Z029218, Z043547, Z093001, and Z029732/Z068035) are shown in Figs. 1 to 4, respectively (in Figs. 1 to 4, thin bars represent genomic DNAs; thick arrows represent candidate genes; and numbers on thin arrows represent the distances between enhancers on T-DNA and the predicted translation initiation sites of the candidate genes).

### (3) Construction of vectors and production of transgenic plants

The open reading frames of candidate genes (At4g31910, At1g04910, At4g35700, and At1g49770) were amplified by PCR using primers described below from a cDNA library prepared from 5-day-old light-grown seedlings (PCR conditions: 30 cycles of 94°C for 30 sec., 60°C for 30 sec., and 72°C for 8 min).
(For At4g31910 amplification)
At4g31910-F:ggggacaagt ttgtacaaaa aagcaggctc gatgcccatg ttatggcga cac (SEQ ID NO:9)
At4g31910-F:ggggaccat ttgtacaaga aagctgggtt tagcaatcaa ggaaatgatt tgaaaagcc (SEQ ID NO:10)
(For At1g04910 amplification)
At1g04910-F :
ggggacaagt ttgtacaaaa aagcaggctc gatggtggcg aaactgtcta tcg (SEQ ID NO:11)
At1g04910-R :ggggaccact ttgtacaaga aagctgggtt taccgttcta atccagtgac cg (SEQ ID NO:12)
(For At4g35700 amplification)
At4g35700-F:
ggggacaagt ttgtacaaaa aagcaggctc gatgagtaat cccgagaagt ctaaaattg (SEQ ID NO:13)
At4g35700-R : ggggaccact ttgtacaaga aagctgggtt cactcgactt tatcatcgtt ctctt (SEQ ID NO:14)
(For At1g049770 amplification)
At1g49770-F :
ggggacaagtttgtacaaaaaagcaggcgtatgactaatgctcaagagttgggg (SEQ ID NO:15)
At1g49770-R : ggggaccactttgtacaagaaagctgggtttgtacaagaaagctgggtttaaagtgaaaagtccaagctaatc (SEQ ID NO:15)

The amplified fragments were cloned into pDONR-207 vector by BP reaction (recombinant reaction between attB and attB) according to the manual of Gateway PCR Cloning System (manufactured by Invitrogen). After sequence confirmation, the inserts were transferred to pBIDAVL-GWR1 using LR Clonase Enzyme Mix (manufactured by Invitrogen) according to the manufacture's instructions. The "reading frame A cassette" of Gateway Vector Conversion System was replaced with the GUS coding region of pBI121, thereby producing the final vector pBillA VL-GWR1. The resulting pBIDAVL-GWR1 cDNA was introduced into *Agrobacterium tumefaciens* GV3101 (pMP90). An *Arabidopsis thaliana* wild type (Col-0) was transformed with this vector by floral dipping technique. Seeds harvested therefrom were placed on sucrose-free GM plates containing 50 mg/l kanamycin and 100 mg/l cefotaxime. Drug resistant strains were transferred to soil and grown in a greenhouse under the light conditions described above.

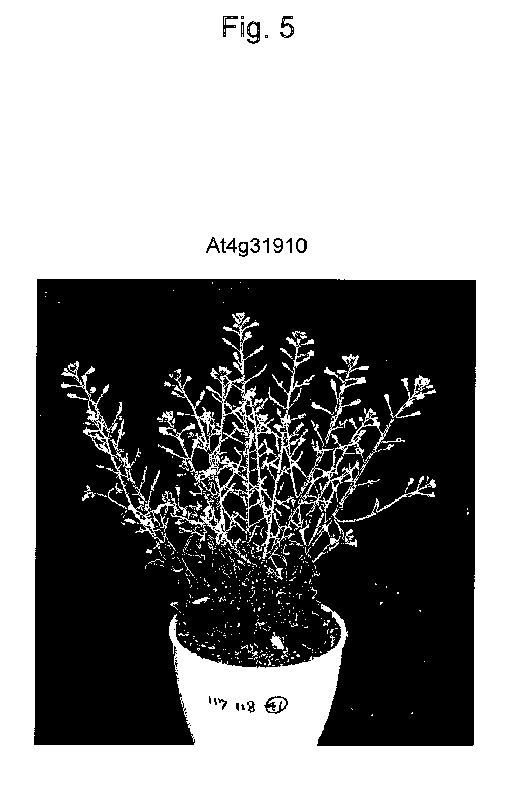

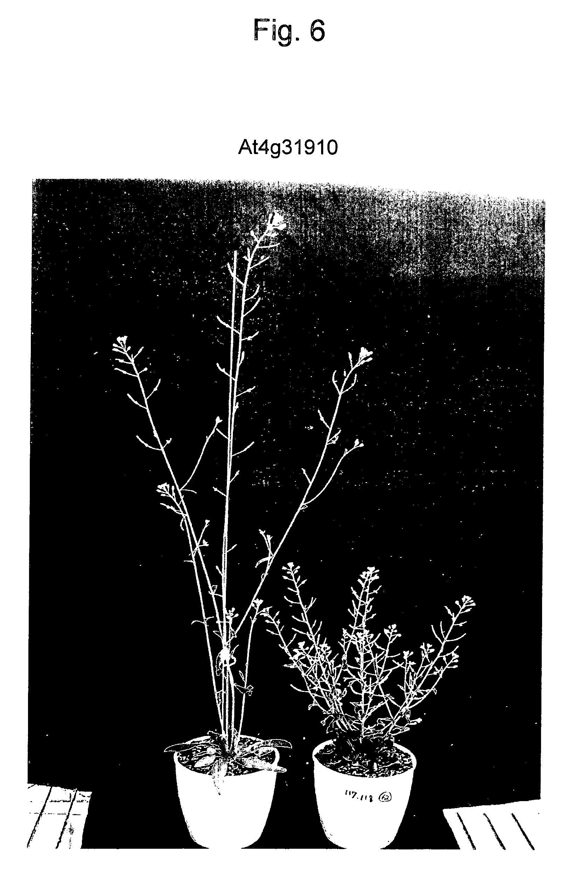

Fig. 5 shows a photograph of an At4g31910 transformant (6-week old). The transformant has a low height, short internodal spacings, and short stems that spread sideways. Fig. 6 is a photograph showing the comparison (6-week old) between an At4g31910 transformant and a wild strain. The At4g31910 transformant is about one-third the height of the wild strain.

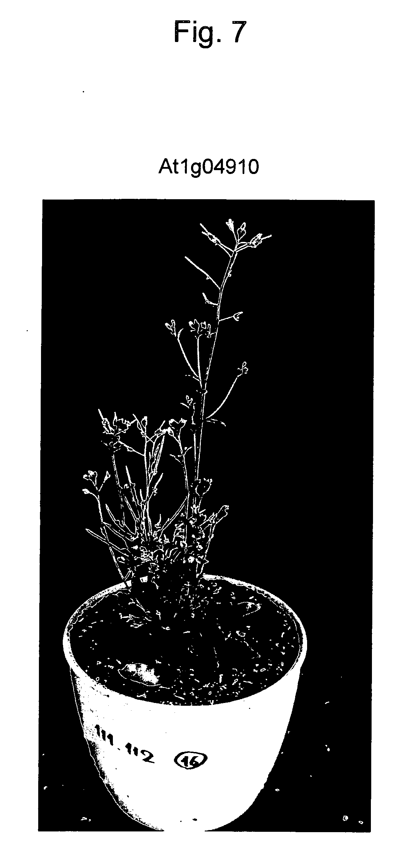

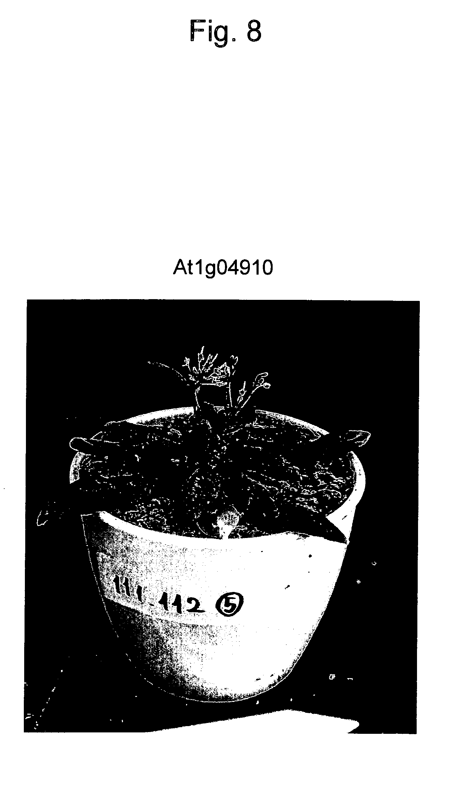

Figs. 7 and 8 respectively show a photograph of an At1g04910 transformant (6-week old). The At1g04910 transformant has a height as low as that of the At4g31910 transformant and has a feature of flowers densely clustered at stem tips in stems grown later. In Fig. 8, the trait is severely exhibited.

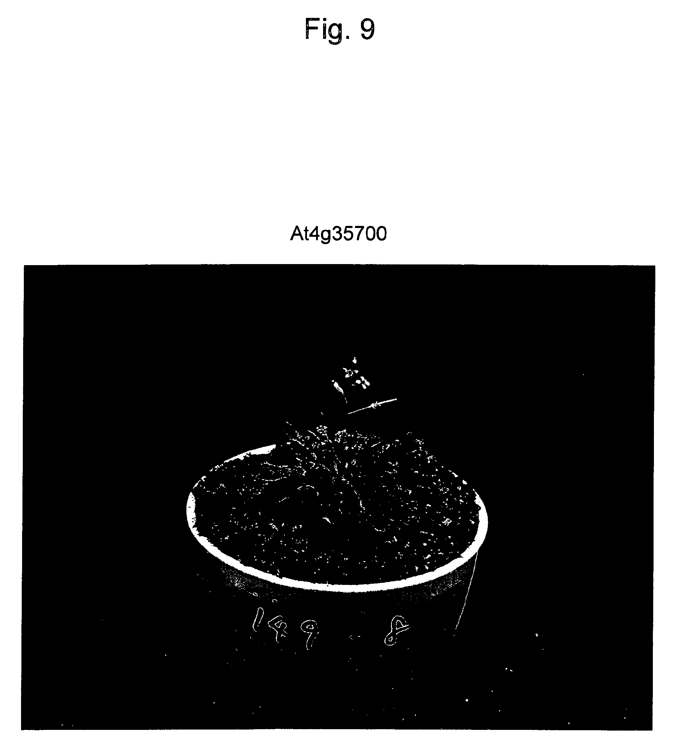

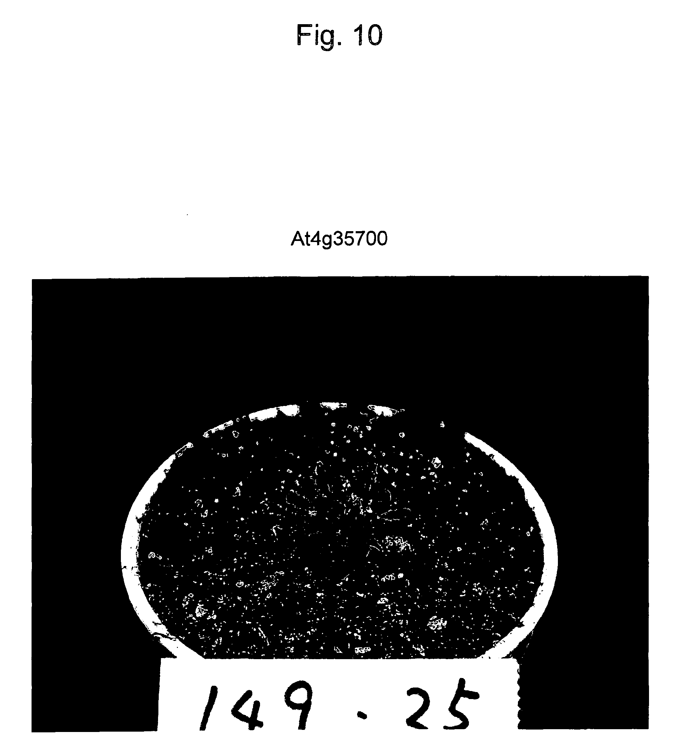

Figs. 9 and 10 respectively show a photograph of an At4g35700 transformant (6-week old). Both of them have a low height and barely extend upward. In Fig. 10, the trait is severely exhibited.

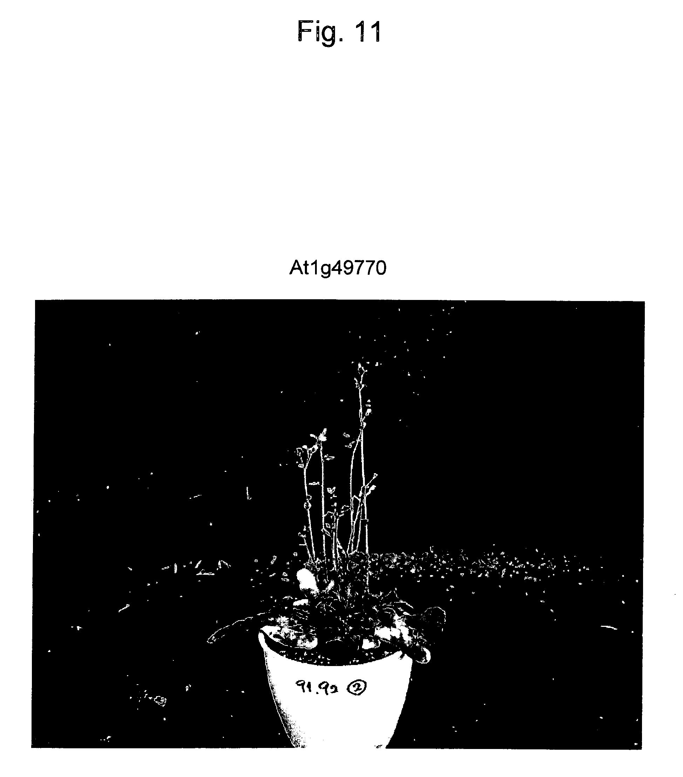

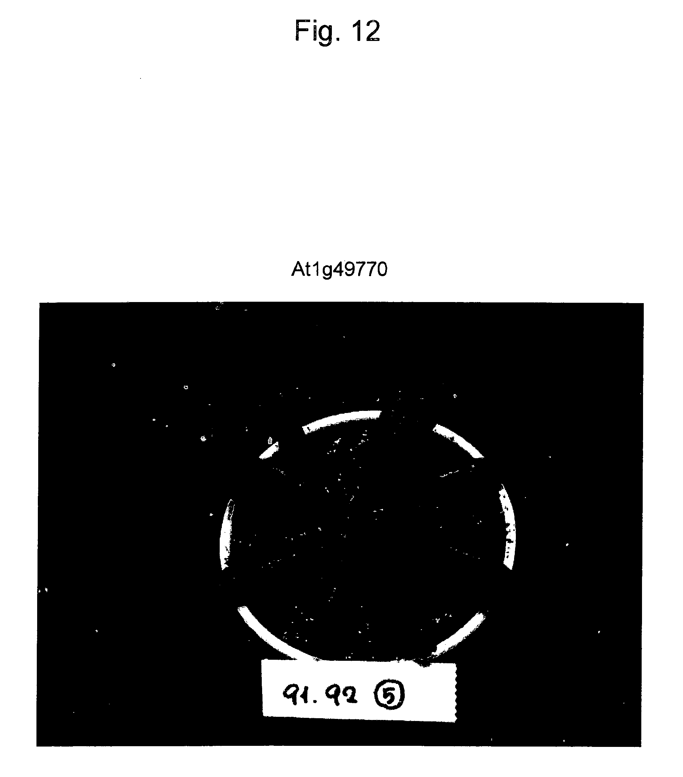

Figs. 11 and 12 respectively show a photograph of an At1g49770 transformant (6-week old). Both of them have a low height and barely extend upward. In Fig. 12, the trait is severely exhibited.

It is noted that the transformants of the genes described above are T₂ generations.

**[Table 1]**

| Strain | Height on 6th week |
|---|---|
| COL-0 | 33 cm |
| At4g31910 | 10 to 15 cm |
| At1g04910 | 3 to 15 cm |
| At4g35700 | 1 to 5 cm |
| At1g49770 | 1 to 20 cm |

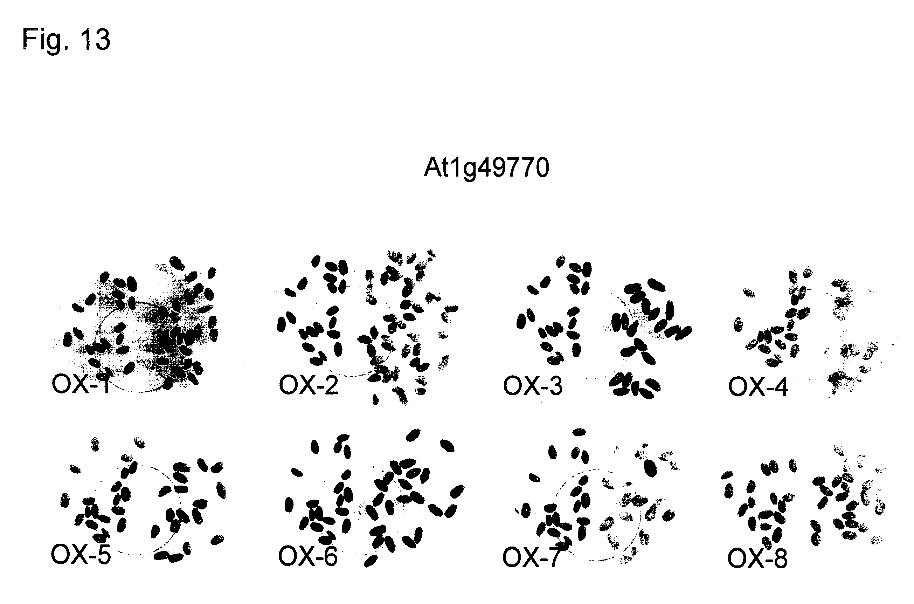

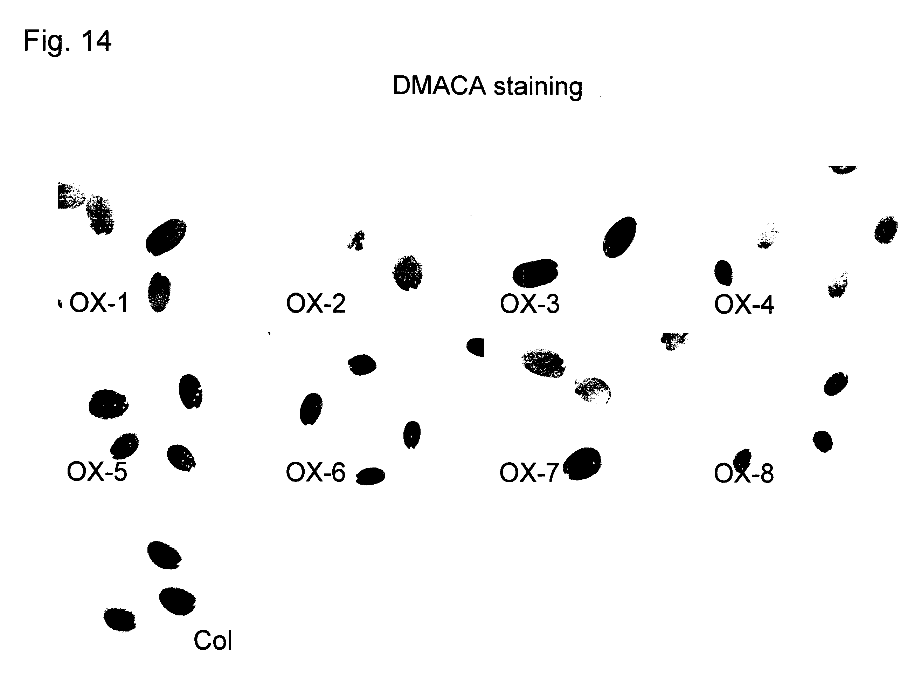

Moreover, the dried seeds of the At1g49770 transformants were dipped for 1 week in a solution of 2% (w/v) DMACA (p-dimethylaminocinnamaldehyde)/3 M HCl/50% (w/v) methanol to observe black staining caused by the bond between tannin and the DMACA. The dried seeds and a result of tannin detection are shown in Figs. 13 and 14, respectively. Wild-type seeds (controls) assumed black color, in which tannin was detected, whereas the At1g49770 transformant seeds were lightly stained, in which tannin contents were reduced.

All publications, patents, and patent applications cited herein are incorporated herein by reference in their entirety.

### Industrial Applicability

The present invention provides a gene having the function of dwarfing a plant body. Because a plant transformed with the gene has a height rendered short, the plant does not fall down by increases in the number of grains and has the effect of being resistant to wind damage such as typhoons.

Thus, the present invention can be utilized in yield enhancement, phenotypic trait diversification, and so on by plant dwarfing.

## Claims

1. A gene encoding a protein of the following (a) or (b):
(a) a protein consisting of the amino acid sequence represented by any of SEQ ID NOs: 2, 4, 6, and 8; or
(b) a protein consisting of an amino acid sequence derived from the amino acid sequence represented by any of SEQ ID NOs: 2, 4, 6, and 8 with the deletion, substitution, or addition of one or several amino acids and having the activity of dwarfing a plant body.

2. A gene comprising DNA of the following (c) or (d):
(c) DNA consisting of the nucleotide sequence represented by any of SEQ ID NOs: 1, 3, 5, and 7; or
(d) DNA hybridizing under stringent conditions to DNA consisting of a nucleotide sequence complementary to DNA consisting of the nucleotide sequence represented by any of SEQ ID NOs: 1, 3, 5, and 7 and encoding a protein having the activity of dwarfing a plant body.

3. A protein of the following (a) or (b):
(a) a protein consisting of the amino acid sequence represented by any of SEQ ID NOs: 2, 4, 6, and 8; or
(b) a protein consisting of an amino acid sequence derived from the amino acid sequence represented by any of SEQ ID NOs: 2, 4, 6, and 8 with the deletion, substitution, or addition of one or several amino acids and having the activity of dwarfing a plant body.

4. A recombinant vector comprising a gene according to claim 1 or 2.

5. A transformed plant with a gene according to claim 1 or 2 or a recombinant vector according to claim 4 introduced therein.

6. The transformed plant according to claim 5, wherein the plant is a plant body, plant organ, plant tissue, or cultured plant cell.

7. The transformed plant according to claim 5 or 6, wherein the plant belongs to any family selected from the group consisting of the families *Brassicaceae, Solanaceae, Poaceae,* and *Leguminosae.*

8. A method for dwarfing a plant body by inducing the overexpression of a gene according to claim 1 or 2 in the plant body.


SEQUENCE LISTING

<110> RIKEN

<110> NEC Soft, Ltd.

<120> A gene associated with dwarf phenotype

<130> PH-2236-PCT

<150> JP 2003-321497

<151> 2003-09-12

<160> 16

<170> PatentIn Ver. 2.1

<210> 1

<211> 1377

<212> DNA

<213> arabidopsis

<220>

<221> CDS

<222> (1)..(1377)

<400> 1

atg ccc atg tta atg gcg aca cgt atc gat ata atc caa aag ctt aat 48

Met Pro Met Leu Met Ala Thr Arg Ile Asp Ile Ile Gln Lys Leu Asn

1               5                   10                  15

gta tat cca agg ttt caa aac cat gac aag aag aaa cta atc act ctc 96
Val Tyr Pro Arg Phe Gln Asn His Asp Lys Lys Lys Leu Ile Thr Leu
            20                  25                  30

tcc aat ttg gac cgt cag tgt cct tta ctc atg tac tct gtc ttc ttc 144
Ser Asn Leu Asp Arg Gln Cys Pro Leu Leu Met Tyr Ser Val Phe Phe
        35                  40                  45

tac aag aat acc aca act cgt gac ttt gac tcc gtc ttc tcc aac ctg 192
Tyr Lys Asn Thr Thr Thr Arg Asp Phe Asp Ser Val Phe Ser Asn Leu
    50                  55                  60

aag ctc ggg ctg gag gag act atg tct gtg tgg tat ccc gcg gca ggg 240
Lys Leu Gly Leu Glu Glu Thr Met Ser Val Trp Tyr Pro Ala Ala Gly
65                  70                  75                  80

aga ctg ggt ttg gac gga ggt ggc tgc aag ctc aac atc cgg tgt aac 288
Arg Leu Gly Leu Asp Gly Gly Gly Cys Lys Leu Asn Ile Arg Cys Asn
                85                  90                  95

gat ggt ggc gca gtc atg gtg gag gcg gtg gcg aca ggt gtc aag ttg 336
Asp Gly Gly Ala Val Met Val Glu Ala Val Ala Thr Gly Val Lys Leu
            100                 105                 110

tcc gag ctt ggt gat ttg act cag tac aat gag ttt tat gag aat tta 384
Ser Glu Leu Gly Asp Leu Thr Gln Tyr Asn Glu Phe Tyr Glu Asn Leu
        115                 120                 125

gtt tac aag cct tcc ttg gat ggt gat ttc tct gtg atg cct ctt gtt 432
Val Tyr Lys Pro Ser Leu Asp Gly Asp Phe Ser Val Met Pro Leu Val

    130                 135                 140

gtt gct cag gtg aca aga ttt gca tgt gga ggt tac tca att gga atc 480
Val Ala Gln Val Thr Arg Phe Ala Cys Gly Gly Tyr Ser Ile Gly Ile
145                 150                 155                 160

ggt aca agc cac tct cta ttt gat gga atc tca gct tac gaa ttc att 528
Gly Thr Ser His Ser Leu Phe Asp Gly Ile Ser Ala Tyr Glu Phe Ile
                165                 170                 175

cac gcg tgg gcc tcc aac tct cac att cac aac aaa tcc aac agc aag 576
His Ala Trp Ala Ser Asn Ser His Ile His Asn Lys Ser Asn Ser Lys
            180                 185                 190

att act aat aaa aag gaa gat gtg gtc atc aaa ccg gtt cat gat cga 624
Ile Thr Asn Lys Lys Glu Asp Val Val Ile Lys Pro Val His Asp Arg
        195                 200                 205

cga aat cta ctg gtt aac cgg gat gct gtc cga gaa acc aat gct gca 672
Arg Asn Leu Leu Val Asn Arg Asp Ala Val Arg Glu Thr Asn Ala Ala
    210                 215                 220

gcc att tgt cat ctg tac cag ttg atc aaa cag gcg atg atg acc tat 720
Ala Ile Cys His Leu Tyr Gln Leu Ile Lys Gln Ala Met Met Thr Tyr
225                 230                 235                 240

cag gag caa aac cgt aac tta gag tta cca gac tct ggt ttt gtg atc 768
Gln Glu Gln Asn Arg Asn Leu Glu Leu Pro Asp Ser Gly Phe Val Ile
                245                 250                 255

aaa acg ttc gag ctt aat ggc gat gcg ata gaa agc atg aag aag aaa 816
Lys Thr Phe Glu Leu Asn Gly Asp Ala Ile Glu Ser Met Lys Lys Lys
            260                 265                 270

tca cta gaa ggg ttc atg tgc tcc tcc ttt gag ttt ctt gct gct cat 864
Ser Leu Glu Gly Phe Met Cys Ser Ser Phe Glu Phe Leu Ala Ala His
        275                 280                 285

ttg tgg aag gca aga aca agg gct tta ggg ttg agg aga gac gcc atg 912
Leu Trp Lys Ala Arg Thr Arg Ala Leu Gly Leu Arg Arg Asp Ala Met
    290                 295                 300

gtg tgt tta caa ttc gca gtg gac ata agg aaa aga acg gag aca ccg 960
Val Cys Leu Gln Phe Ala Val Asp Ile Arg Lys Arg Thr Glu Thr Pro
305                 310                 315                 320

ctg cca gaa ggg ttt tcc ggc aac gca tac gtg ctt gcc tcg gtg gca 1008
Leu Pro Glu Gly Phe Ser Gly Asn Ala Tyr Val Leu Ala Ser Val Ala
                325                 330                 335

tcc acc gcc aga gaa tta ctt gaa gag cta aca ctc gag tca ata gtc 1056
Ser Thr Ala Arg Glu Leu Leu Glu Glu Leu Thr Leu Glu Ser Ile Val
            340                 345                 350

aac aag atc aga gaa gcc aag aaa tca att gac caa ggt tac ata aac 1104
Asn Lys Ile Arg Glu Ala Lys Lys Ser Ile Asp Gln Gly Tyr Ile Asn
        355                 360                 365

tct tac atg gaa gca ctc gga ggt agt aat gac gga aat ctc cct cct 1152
Ser Tyr Met Glu Ala Leu Gly Gly Ser Asn Asp Gly Asn Leu Pro Pro

    370                 375                 380

ctc aaa gag cta acc cta atc tcc gac tgg aca aaa atg cca ttt cac 1200
Leu Lys Glu Leu Thr Leu Ile Ser Asp Trp Thr Lys Met Pro Phe His
385                 390                 395                 400

aat gtt ggc ttt ggc aac ggc ggc gag cca gcg gat tac atg gcc cca 1248
Asn Val Gly Phe Gly Asn Gly Gly Glu Pro Ala Asp Tyr Met Ala Pro
                405                 410                 415

ctg tgt cca ccg gtg cca caa gtt gct tat ttc atg aag aac cct aaa 1296
Leu Cys Pro Pro Val Pro Gln Val Ala Tyr Phe Met Lys Asn Pro Lys
            420                 425                 430

gat gcc aaa ggt gtt ctt gtg agg att ggc ttg gac cca cga gat gtt 1344
Asp Ala Lys Gly Val Leu Val Arg Ile Gly Leu Asp Pro Arg Asp Val
        435                 440                 445

aat ggc ttt tca aat cat ttc ctt gat tgc taa 1377
Asn Gly Phe Ser Asn His Phe Leu Asp Cys
    450                 455

<210> 2
<211> 458
<212> PRT
<213> arabidopsis

<400> 2
Met Pro Met Leu Met Ala Thr Arg Ile Asp Ile Ile Gln Lys Leu Asn
1               5                   10                  15

Val Tyr Pro Arg Phe Gln Asn His Asp Lys Lys Lys Leu Ile Thr Leu
            20                  25                  30
Ser Asn Leu Asp Arg Gln Cys Pro Leu Leu Met Tyr Ser Val Phe Phe
        35                  40                  45
Tyr Lys Asn Thr Thr Thr Arg Asp Phe Asp Ser Val Phe Ser Asn Leu
    50                  55                  60
Lys Leu Gly Leu Glu Glu Thr Met Ser Val Trp Tyr Pro Ala Ala Gly
65                  70                  75                  80
Arg Leu Gly Leu Asp Gly Gly Gly Cys Lys Leu Asn Ile Arg Cys Asn
                85                  90                  95
Asp Gly Gly Ala Val Met Val Glu Ala Val Ala Thr Gly Val Lys Leu
            100                 105                 110
Ser Glu Leu Gly Asp Leu Thr Gln Tyr Asn Glu Phe Tyr Glu Asn Leu
        115                 120                 125
Val Tyr Lys Pro Ser Leu Asp Gly Asp Phe Ser Val Met Pro Leu Val
    130                 135                 140
Val Ala Gln Val Thr Arg Phe Ala Cys Gly Gly Tyr Ser Ile Gly Ile
145                 150                 155                 160
Gly Thr Ser His Ser Leu Phe Asp Gly Ile Ser Ala Tyr Glu Phe Ile
                165                 170                 175
His Ala Trp Ala Ser Asn Ser His Ile His Asn Lys Ser Asn Ser Lys
            180                 185                 190
Ile Thr Asn Lys Lys Glu Asp Val Val Ile Lys Pro Val His Asp Arg
        195                 200                 205
Arg Asn Leu Leu Val Asn Arg Asp Ala Val Arg Glu Thr Asn Ala Ala
    210                 215                 220
Ala Ile Cys His Leu Tyr Gln Leu Ile Lys Gln Ala Met Met Thr Tyr
225                 230                 235                 240
Gln Glu Gln Asn Arg Asn Leu Glu Leu Pro Asp Ser Gly Phe Val Ile
                245                 250                 255

Lys Thr Phe Glu Leu Asn Gly Asp Ala Ile Glu Ser Met Lys Lys Lys
            260                 265                 270
Ser Leu Glu Gly Phe Met Cys Ser Ser Phe Glu Phe Leu Ala Ala His
        275                 280                 285
Leu Trp Lys Ala Arg Thr Arg Ala Leu Gly Leu Arg Arg Asp Ala Met
    290                 295                 300
Val Cys Leu Gln Phe Ala Val Asp Ile Arg Lys Arg Thr Glu Thr Pro
305                 310                 315                 320
Leu Pro Glu Gly Phe Ser Gly Asn Ala Tyr Val Leu Ala Ser Val Ala
                325                 330                 335
Ser Thr Ala Arg Glu Leu Leu Glu Glu Leu Thr Leu Glu Ser Ile Val
            340                 345                 350
Asn Lys Ile Arg Glu Ala Lys Lys Ser Ile Asp Gln Gly Tyr Ile Asn
        355                 360                 365
Ser Tyr Met Glu Ala Leu Gly Gly Ser Asn Asp Gly Asn Leu Pro Pro
    370                 375                 380
Leu Lys Glu Leu Thr Leu Ile Ser Asp Trp Thr Lys Met Pro Phe His
385                 390                 395                 400
Asn Val Gly Phe Gly Asn Gly Gly Glu Pro Ala Asp Tyr Met Ala Pro
                405                 410                 415
Leu Cys Pro Pro Val Pro Gln Val Ala Tyr Phe Met Lys Asn Pro Lys
            420                 425                 430
Asp Ala Lys Gly Val Leu Val Arg Ile Gly Leu Asp Pro Arg Asp Val
        435                 440                 445
Asn Gly Phe Ser Asn His Phe Leu Asp Cys
    450                 455

<210> 3
<211> 1560
<212> DNA

<213> arabidopsis

<220>
<221> CDS
<222> (1)..(1560)

<400> 3
atg cga agg tta ggt cac cac cga ctt cat ggg aag acg gga gga gtt     48
Met Arg Arg Leu Gly His His Arg Leu His Gly Lys Thr Gly Gly Val
1               5                   10                  15

gga aca aag ggg atg gtg gcg aaa ctg tct atc gga gtc att gtt cta     96
Gly Thr Lys Gly Met Val Ala Lys Leu Ser Ile Gly Val Ile Val Leu
            20                  25                  30

ctg atc tgt acg ctc tcg ctg ctt ttc tcg gct aac atc ggt agc aat     144
Leu Ile Cys Thr Leu Ser Leu Leu Phe Ser Ala Asn Ile Gly Ser Asn
        35                  40                  45

cgc gaa ccg acc cgt cca tct aag ata aac gtg gaa gag ctc tgg gag     192
Arg Glu Pro Thr Arg Pro Ser Lys Ile Asn Val Glu Glu Leu Trp Glu
    50                  55                  60

agt gct aaa tct ggt ggt tgg aga ccg tct tct gca cca cga tct gac     240
Ser Ala Lys Ser Gly Gly Trp Arg Pro Ser Ser Ala Pro Arg Ser Asp
65                  70                  75                  80

tgg cct cct cct acg aag gag aca aat ggt tat ctc cgt gtt cga tgt     288
Trp Pro Pro Pro Thr Lys Glu Thr Asn Gly Tyr Leu Arg Val Arg Cys
                85                  90                  95

aat ggt ggt ttg aat cag caa cgt agt gcg ata tgt aat gca gta ctt 336
Asn Gly Gly Leu Asn Gln Gln Arg Ser Ala Ile Cys Asn Ala Val Leu
            100                 105                 110

gct gcg cgg atc atg aat gct aca ctt gtg ttg ccc gaa tta gat gca 384
Ala Ala Arg Ile Met Asn Ala Thr Leu Val Leu Pro Glu Leu Asp Ala
        115                 120                 125

aac tcg ttt tgg cat gat gac agt ggt ttt caa ggt atc tat gac gtt 432
Asn Ser Phe Trp His Asp Asp Ser Gly Phe Gln Gly Ile Tyr Asp Val
    130                 135                 140

gaa cat ttt att gag aca ctg aag tat gat gtt aag att gtg gga aag 480
Glu His Phe Ile Glu Thr Leu Lys Tyr Asp Val Lys Ile Val Gly Lys
145                 150                 155                 160

atc ccc gat gtt cac aaa aat ggg aaa acc aag aag ata aaa gct ttt 528
Ile Pro Asp Val His Lys Asn Gly Lys Thr Lys Lys Ile Lys Ala Phe
                165                 170                 175

cag att cgt cct cct cga gat gct cct att gag tgg tac ctg aca act 576
Gln Ile Arg Pro Pro Arg Asp Ala Pro Ile Glu Trp Tyr Leu Thr Thr
            180                 185                 190

gct cta aag gca atg agg gaa cat agt gct atc tat ctt acc cct ttt 624
Ala Leu Lys Ala Met Arg Glu His Ser Ala Ile Tyr Leu Thr Pro Phe
        195                 200                 205

tca cat cgt tta gcg gag gaa att gac aat cct gaa tat caa agg tta 672

Ser His Arg Leu Ala Glu Glu Ile Asp Asn Pro Glu Tyr Gln Arg Leu
    210                 215                 220

agg tgc aga gtg aac tac cat gct ttg aga ttc aag cca cac atc atg 720
Arg Cys Arg Val Asn Tyr His Ala Leu Arg Phe Lys Pro His Ile Met
225                 230                 235                 240

aag ttg agt gaa tct att gtt gat aaa ctc cgg tca cag ggc cac ttc 768
Lys Leu Ser Glu Ser Ile Val Asp Lys Leu Arg Ser Gln Gly His Phe
                245                 250                 255

atg tcg atc cat ctt cgt ttt gaa atg gat atg ttg gca ttt gcg ggg 816
Met Ser Ile His Leu Arg Phe Glu Met Asp Met Leu Ala Phe Ala Gly
            260                 265                 270

tgc ttt gac ata ttt aac cct gag gaa cag aag ata ctt agg aag tac 864
Cys Phe Asp Ile Phe Asn Pro Glu Glu Gln Lys Ile Leu Arg Lys Tyr
        275                 280                 285

cgt aaa gaa aat ttt gca gat aaa agg ctt att tac aac gaa aga cgg 912
Arg Lys Glu Asn Phe Ala Asp Lys Arg Leu Ile Tyr Asn Glu Arg Arg
    290                 295                 300

gct atc ggg aag tgt cca ctg acc cct gag gag gta ggt ctt ata tta 960
Ala Ile Gly Lys Cys Pro Leu Thr Pro Glu Glu Val Gly Leu Ile Leu
305                 310                 315                 320

cga gca atg aga ttc gac aac tca aca agg ata tac cta gca gcc ggt 1008
Arg Ala Met Arg Phe Asp Asn Ser Thr Arg Ile Tyr Leu Ala Ala Gly
                325                 330                 335

gaa ctt ttt ggc ggg gaa cag ttc atg aaa ccg ttt cga aca tta ttc 1056
Glu Leu Phe Gly Gly Glu Gln Phe Met Lys Pro Phe Arg Thr Leu Phe
            340                 345                 350

cca cgg ctt gat aac cat agc tcg gta gac ccc tct gag gag ctg tca 1104
Pro Arg Leu Asp Asn His Ser Ser Val Asp Pro Ser Glu Glu Leu Ser
        355                 360                 365

gca acc tca cag gga tta ata gga tca gct gtg gat tac atg gtt tgt 1152
Ala Thr Ser Gln Gly Leu Ile Gly Ser Ala Val Asp Tyr Met Val Cys
    370                 375                 380

ctt ctc tcc gac att ttt atg cca aca tat gat gga ccg agc aat ttc 1200
Leu Leu Ser Asp Ile Phe Met Pro Thr Tyr Asp Gly Pro Ser Asn Phe
385                 390                 395                 400

gcc aac aat ctc tta ggt cat cgc ctt tac tat ggt ttc cgt acc aca 1248
Ala Asn Asn Leu Leu Gly His Arg Leu Tyr Tyr Gly Phe Arg Thr Thr
                405                 410                 415

atc aga cca gac aga aaa gca tta gct cca att ttc ata gct aga gag 1296
Ile Arg Pro Asp Arg Lys Ala Leu Ala Pro Ile Phe Ile Ala Arg Glu
            420                 425                 430

aag ggg aaa agg gct ggc ttc gaa gag gca gtg aga cga gta atg ctg 1344
Lys Gly Lys Arg Ala Gly Phe Glu Glu Ala Val Arg Arg Val Met Leu
        435                 440                 445

aaa aca aac ttc gga ggg cct cac aag cgt gta tct cct gaa tcg ttt 1392

Lys Thr Asn Phe Gly Gly Pro His Lys Arg Val Ser Pro Glu Ser Phe
    450                 455                 460

tat aca aat tca tgg cct gaa tgt ttc tgt cag atg aat ccg aag aag    1440
Tyr Thr Asn Ser Trp Pro Glu Cys Phe Cys Gln Met Asn Pro Lys Lys
465                 470                 475                 480

tct agt gat aaa tgc ccg cca aat aat gtt ata gag att ctt gac agc    1488
Ser Ser Asp Lys Cys Pro Pro Asn Asn Val Ile Glu Ile Leu Asp Ser
                485                 490                 495

cgg tta gag agt atc aga gat cca gat tca act tct cag acg aat tcc    1536
Arg Leu Glu Ser Ile Arg Asp Pro Asp Ser Thr Ser Gln Thr Asn Ser
            500                 505                 510

acg gtc act gga tta gaa cgg taa                                    1560
Thr Val Thr Gly Leu Glu Arg
        515                 520

<210> 4
<211> 519
<212> PRT
<213> arabidopsis

<400> 4
Met Arg Arg Leu Gly His His Arg Leu His Gly Lys Thr Gly Gly Val
  1               5                  10                  15
Gly Thr Lys Gly Met Val Ala Lys Leu Ser Ile Gly Val Ile Val Leu
             20                  25                  30
Leu Ile Cys Thr Leu Ser Leu Leu Phe Ser Ala Asn Ile Gly Ser Asn

        35                  40                  45
Arg Glu Pro Thr Arg Pro Ser Lys Ile Asn Val Glu Glu Leu Trp Glu
    50                  55                  60
Ser Ala Lys Ser Gly Gly Trp Arg Pro Ser Ser Ala Pro Arg Ser Asp
65                  70                  75                  80
Trp Pro Pro Pro Thr Lys Glu Thr Asn Gly Tyr Leu Arg Val Arg Cys
                85                  90                  95
Asn Gly Gly Leu Asn Gln Gln Arg Ser Ala Ile Cys Asn Ala Val Leu
            100                 105                 110
Ala Ala Arg Ile Met Asn Ala Thr Leu Val Leu Pro Glu Leu Asp Ala
        115                 120                 125
Asn Ser Phe Trp His Asp Asp Ser Gly Phe Gln Gly Ile Tyr Asp Val
    130                 135                 140
Glu His Phe Ile Glu Thr Leu Lys Tyr Asp Val Lys Ile Val Gly Lys
145                 150                 155                 160
Ile Pro Asp Val His Lys Asn Gly Lys Thr Lys Lys Ile Lys Ala Phe
                165                 170                 175
Gln Ile Arg Pro Pro Arg Asp Ala Pro Ile Glu Trp Tyr Leu Thr Thr
            180                 185                 190
Ala Leu Lys Ala Met Arg Glu His Ser Ala Ile Tyr Leu Thr Pro Phe
        195                 200                 205
Ser His Arg Leu Ala Glu Glu Ile Asp Asn Pro Glu Tyr Gln Arg Leu
    210                 215                 220
Arg Cys Arg Val Asn Tyr His Ala Leu Arg Phe Lys Pro His Ile Met
225                 230                 235                 240
Lys Leu Ser Glu Ser Ile Val Asp Lys Leu Arg Ser Gln Gly His Phe
                245                 250                 255
Met Ser Ile His Leu Arg Phe Glu Met Asp Met Leu Ala Phe Ala Gly
            260                 265                 270
Cys Phe Asp Ile Phe Asn Pro Glu Glu Gln Lys Ile Leu Arg Lys Tyr

        275                 280                 285
Arg Lys Glu Asn Phe Ala Asp Lys Arg Leu Ile Tyr Asn Glu Arg Arg
    290                 295                 300
Ala Ile Gly Lys Cys Pro Leu Thr Pro Glu Glu Val Gly Leu Ile Leu
305                 310                 315                 320
Arg Ala Met Arg Phe Asp Asn Ser Thr Arg Ile Tyr Leu Ala Ala Gly
                325                 330                 335
Glu Leu Phe Gly Gly Glu Gln Phe Met Lys Pro Phe Arg Thr Leu Phe
            340                 345                 350
Pro Arg Leu Asp Asn His Ser Ser Val Asp Pro Ser Glu Glu Leu Ser
        355                 360                 365
Ala Thr Ser Gln Gly Leu Ile Gly Ser Ala Val Asp Tyr Met Val Cys
    370                 375                 380
Leu Leu Ser Asp Ile Phe Met Pro Thr Tyr Asp Gly Pro Ser Asn Phe
385                 390                 395                 400
Ala Asn Asn Leu Leu Gly His Arg Leu Tyr Tyr Gly Phe Arg Thr Thr
                405                 410                 415
Ile Arg Pro Asp Arg Lys Ala Leu Ala Pro Ile Phe Ile Ala Arg Glu
            420                 425                 430
Lys Gly Lys Arg Ala Gly Phe Glu Glu Ala Val Arg Arg Val Met Leu
        435                 440                 445
Lys Thr Asn Phe Gly Gly Pro His Lys Arg Val Ser Pro Glu Ser Phe
    450                 455                 460
Tyr Thr Asn Ser Trp Pro Glu Cys Phe Cys Gln Met Asn Pro Lys Lys
465                 470                 475                 480
Ser Ser Asp Lys Cys Pro Pro Asn Asn Val Ile Glu Ile Leu Asp Ser
                485                 490                 495
Arg Leu Glu Ser Ile Arg Asp Pro Asp Ser Thr Ser Gln Thr Asn Ser
            500                 505                 510
Thr Val Thr Gly Leu Glu Arg

        515

<210> 5
<211> 828
<212> DNA
<213> arabidopsis

<220>
<221> CDS
<222> (1)..(828)

<400> 5
atg agt aat ccc gag aag tct aaa att gct gag gaa gag aat gag aat     48
Met Ser Asn Pro Glu Lys Ser Lys Ile Ala Glu Glu Glu Asn Glu Asn
1               5                   10                  15

ttt gac gag agt gat tat ctt tgg agc gat gaa gaa gga gac ata cgt     96
Phe Asp Glu Ser Asp Tyr Leu Trp Ser Asp Glu Glu Gly Asp Ile Arg
            20                  25                  30

gaa att gtt ctt ggc ctt cct gca cta aga ctt agt gag agt ctc aat     144
Glu Ile Val Leu Gly Leu Pro Ala Leu Arg Leu Ser Glu Ser Leu Asn
        35                  40                  45

gtg gat gaa agc atc cgt aac gaa gcc gtt tcc gcg gcg aag ttg atc     192
Val Asp Glu Ser Ile Arg Asn Glu Ala Val Ser Ala Ala Lys Leu Ile
    50                  55                  60

gtg gca gcc gcc aag gaa gcc aca ggg aat aca gaa caa agt gct agt     240
Val Ala Ala Ala Lys Glu Ala Thr Gly Asn Thr Glu Gln Ser Ala Ser

65                  70                  75                  80

ggt acc ggt gac gtt agt gga act cca gaa atc ata aaa gtt ggg aaa     288
Gly Thr Gly Asp Val Ser Gly Thr Pro Glu Ile Ile Lys Val Gly Lys
                85                  90                  95

ccc cgt gta tta ttg gaa gcg ctg gcc gaa gca tct ggt tgt acc cct     336
Pro Arg Val Leu Leu Glu Ala Leu Ala Glu Ala Ser Gly Cys Thr Pro
            100                 105                 110

att ggg gtg acc act act gaa gga gag gta gtc aag aaa ccc cgt aag     384
Ile Gly Val Thr Thr Thr Glu Gly Glu Val Val Lys Lys Pro Arg Lys
        115                 120                 125

aaa ggt tct act cag ttg aca cat ctg ccg caa ggc cct cct atg tgt     432
Lys Gly Ser Thr Gln Leu Thr His Leu Pro Gln Gly Pro Pro Met Cys
    130                 135                 140

aat gtt tgt ggg aag ggc ttt gct tct tgg aag gct gtt ttt gga cat     480
Asn Val Cys Gly Lys Gly Phe Ala Ser Trp Lys Ala Val Phe Gly His
145                 150                 155                 160

cta agg cag cat aag gat cga ggg tat cta ggt ttt ctt cct ccg cca     528
Leu Arg Gln His Lys Asp Arg Gly Tyr Leu Gly Phe Leu Pro Pro Pro
                165                 170                 175

aca ttc aat gct gct gag gaa gga ttt ggt gga gtt gct act gct tca     576
Thr Phe Asn Ala Ala Glu Glu Gly Phe Gly Gly Val Ala Thr Ala Ser
            180                 185                 190

acc ggt ggt gca gta gcc act gct att tcc gtt ggt gaa gtc cct act 624
Thr Gly Gly Ala Val Ala Thr Ala Ile Ser Val Gly Glu Val Pro Thr
        195                 200                 205

gct tct tcc gct ggt gga ctc gct acg gct tct tcc gat ggt gaa ggg 672
Ala Ser Ser Ala Gly Gly Leu Ala Thr Ala Ser Ser Asp Gly Glu Gly
    210                 215                 220

ctt gtt tca agt atc gga agg gcg agg att gac tta aat gcc gat cca 720
Leu Val Ser Ser Ile Gly Arg Ala Arg Ile Asp Leu Asn Ala Asp Pro
225                 230                 235                 240

gtc gag gag gaa gac aag gta att gga gtt gga tct aag cga aaa ttt 768
Val Glu Glu Glu Asp Lys Val Ile Gly Val Gly Ser Lys Arg Lys Phe
                245                 250                 255

gat ctg aat agg tcc ccc cca cca gac ggt gat gaa gag aac gat gat 816
Asp Leu Asn Arg Ser Pro Pro Pro Asp Gly Asp Glu Glu Asn Asp Asp
            260                 265                 270

aaa gtc gag tga 828
Lys Val Glu
        275

<210> 6
<211> 275
<212> PRT
<213> arabidopsis

<400> 6

Met Ser Asn Pro Glu Lys Ser Lys Ile Ala Glu Glu Glu Asn Glu Asn
1               5                   10                  15
Phe Asp Glu Ser Asp Tyr Leu Trp Ser Asp Glu Glu Gly Asp Ile Arg
            20                  25                  30
Glu Ile Val Leu Gly Leu Pro Ala Leu Arg Leu Ser Glu Ser Leu Asn
        35                  40                  45
Val Asp Glu Ser Ile Arg Asn Glu Ala Val Ser Ala Ala Lys Leu Ile
    50                  55                  60
Val Ala Ala Ala Lys Glu Ala Thr Gly Asn Thr Glu Gln Ser Ala Ser
65                  70                  75                  80
Gly Thr Gly Asp Val Ser Gly Thr Pro Glu Ile Ile Lys Val Gly Lys
                85                  90                  95
Pro Arg Val Leu Leu Glu Ala Leu Ala Glu Ala Ser Gly Cys Thr Pro
            100                 105                 110
Ile Gly Val Thr Thr Thr Glu Gly Glu Val Val Lys Lys Pro Arg Lys
        115                 120                 125
Lys Gly Ser Thr Gln Leu Thr His Leu Pro Gln Gly Pro Pro Met Cys
    130                 135                 140
Asn Val Cys Gly Lys Gly Phe Ala Ser Trp Lys Ala Val Phe Gly His
145                 150                 155                 160
Leu Arg Gln His Lys Asp Arg Gly Tyr Leu Gly Phe Leu Pro Pro Pro
                165                 170                 175
Thr Phe Asn Ala Ala Glu Glu Gly Phe Gly Gly Val Ala Thr Ala Ser
            180                 185                 190
Thr Gly Gly Ala Val Ala Thr Ala Ile Ser Val Gly Glu Val Pro Thr
        195                 200                 205
Ala Ser Ser Ala Gly Gly Leu Ala Thr Ala Ser Ser Asp Gly Glu Gly
    210                 215                 220
Leu Val Ser Ser Ile Gly Arg Ala Arg Ile Asp Leu Asn Ala Asp Pro
225                 230                 235                 240

Val Glu Glu Glu Asp Lys Val Ile Gly Val Gly Ser Lys Arg Lys Phe
                245                 250                 255
Asp Leu Asn Arg Ser Pro Pro Pro Asp Gly Asp Glu Glu Asn Asp Asp
            260                 265                 270
Lys Val Glu
        275

<210> 7
<211> 927
<212> DNA
<213> arabidopsis

<220>
<221> CDS
<222> (1)..(927)

<400> 7
atg act aat gct caa gag ttg ggg caa gag ggt ttt atg tgg ggc ata 48
Met Thr Asn Ala Gln Glu Leu Gly Gln Glu Gly Phe Met Trp Gly Ile
1               5                   10                  15

tcc aat tct gat gat tcc gga ggt ggt tgc aaa aga atc gag aaa gaa 96
Ser Asn Ser Asp Asp Ser Gly Gly Gly Cys Lys Arg Ile Glu Lys Glu
            20                  25                  30

ccg ctt cca tca cat ccg tct cat ccg tcg cca gag atc cag act acg 144
Pro Leu Pro Ser His Pro Ser His Pro Ser Pro Glu Ile Gln Thr Thr
        35                  40                  45

acg gtt aaa aag gga aaa aag aga acg aaa aga aat gat aaa aac cat 192

Thr Val Lys Lys Gly Lys Lys Arg Thr Lys Arg Asn Asp Lys Asn His
    50                  55                  60

gaa gaa gaa tca cct gat cat gaa ata cat ata tgg acc gaa aga gaa 240
Glu Glu Glu Ser Pro Asp His Glu Ile His Ile Trp Thr Glu Arg Glu
65                  70                  75                  80

agg aga aag aag atg agg gac atg ttt tct aaa tta cat gct ttg ctt 288
Arg Arg Lys Lys Met Arg Asp Met Phe Ser Lys Leu His Ala Leu Leu
                85                  90                  95

ccc caa ctt cct cct aag gca gac aaa tca aca att gta gat gaa gca 336
Pro Gln Leu Pro Pro Lys Ala Asp Lys Ser Thr Ile Val Asp Glu Ala
            100                 105                 110

gtg agt tca atc aaa tcc ctt gaa caa act ttg caa aaa ctt gaa atg 384
Val Ser Ser Ile Lys Ser Leu Glu Gln Thr Leu Gln Lys Leu Glu Met
        115                 120                 125

caa aag ctt gag aag ctt cag tat tct tct gcc tca aca aac aca act 432
Gln Lys Leu Glu Lys Leu Gln Tyr Ser Ser Ala Ser Thr Asn Thr Thr
    130                 135                 140

cct act aca act ttt gct tat gct cca tca tca tcc tct tct cca aca 480
Pro Thr Thr Thr Phe Ala Tyr Ala Pro Ser Ser Ser Ser Ser Pro Thr
145                 150                 155                 160

gct ctc ctc aca cca ata tca aac cat ccc ata gac gcc acc gca aca 528
Ala Leu Leu Thr Pro Ile Ser Asn His Pro Ile Asp Ala Thr Ala Thr
                165                 170                 175

gat tct tac cct cgc gca gct ttc ttg gcc gat cag gta tct tct tcc 576
Asp Ser Tyr Pro Arg Ala Ala Phe Leu Ala Asp Gln Val Ser Ser Ser
            180                 185                 190

agt gcc gct gct gcg aac ctg cct tac cct tgt aat gat ccg atc gtg 624
Ser Ala Ala Ala Ala Asn Leu Pro Tyr Pro Cys Asn Asp Pro Ile Val
        195                 200                 205

aat ttc gat act tgg tcc tca cgt aac gtg gtg ctg act atc tgt ggg 672
Asn Phe Asp Thr Trp Ser Ser Arg Asn Val Val Leu Thr Ile Cys Gly
    210                 215                 220

aat gaa gct ttc ttc aat ttg tgt gtc cct aaa cac aaa cca ggg gtt 720
Asn Glu Ala Phe Phe Asn Leu Cys Val Pro Lys His Lys Pro Gly Val
225                 230                 235                 240

ttc act tct gtt tgt tac ttg ttt gag aag tac aac atg gag gtt ttg 768
Phe Thr Ser Val Cys Tyr Leu Phe Glu Lys Tyr Asn Met Glu Val Leu
                245                 250                 255

ttt gct aat gtc tcc tct aat gtt ttc tgg agc acc tac gtg atc caa 816
Phe Ala Asn Val Ser Ser Asn Val Phe Trp Ser Thr Tyr Val Ile Gln
            260                 265                 270

gca cag gta aat ccg agt tgt gag aat cag tta ctg gga aat ggt ctt 864
Ala Gln Val Asn Pro Ser Cys Glu Asn Gln Leu Leu Gly Asn Gly Leu
        275                 280                 285

gga gtt gta gac gtt ttc aag caa gtt tct caa gaa ctg gtg tta tat 912

Gly Val Val Asp Val Phe Lys Gln Val Ser Gln Glu Leu Val Leu Tyr
    290                 295                 300

ttt tca tct cta taa                                                927
Phe Ser Ser Leu
305

<210> 8
<211> 308
<212> PRT
<213> arabidopsis

<400> 8
Met Thr Asn Ala Gln Glu Leu Gly Gln Glu Gly Phe Met Trp Gly Ile
 1               5                   10                  15
Ser Asn Ser Asp Asp Ser Gly Gly Gly Cys Lys Arg Ile Glu Lys Glu
            20                  25                  30
Pro Leu Pro Ser His Pro Ser His Pro Ser Pro Glu Ile Gln Thr Thr
        35                  40                  45
Thr Val Lys Lys Gly Lys Lys Arg Thr Lys Arg Asn Asp Lys Asn His
    50                  55                  60
Glu Glu Glu Ser Pro Asp His Glu Ile His Ile Trp Thr Glu Arg Glu
65                  70                  75                  80
Arg Arg Lys Lys Met Arg Asp Met Phe Ser Lys Leu His Ala Leu Leu
                85                  90                  95
Pro Gln Leu Pro Pro Lys Ala Asp Lys Ser Thr Ile Val Asp Glu Ala
            100                 105                 110
Val Ser Ser Ile Lys Ser Leu Glu Gln Thr Leu Gln Lys Leu Glu Met
        115                 120                 125
Gln Lys Leu Glu Lys Leu Gln Tyr Ser Ser Ala Ser Thr Asn Thr Thr

130                 135                 140

Pro Thr Thr Thr Phe Ala Tyr Ala Pro Ser Ser Ser Ser Ser Pro Thr

145                 150                 155                 160

Ala Leu Leu Thr Pro Ile Ser Asn His Pro Ile Asp Ala Thr Ala Thr

                165                 170                 175

Asp Ser Tyr Pro Arg Ala Ala Phe Leu Ala Asp Gln Val Ser Ser Ser

            180                 185                 190

Ser Ala Ala Ala Ala Asn Leu Pro Tyr Pro Cys Asn Asp Pro Ile Val

        195                 200                 205

Asn Phe Asp Thr Trp Ser Ser Arg Asn Val Val Leu Thr Ile Cys Gly

    210                 215                 220

Asn Glu Ala Phe Phe Asn Leu Cys Val Pro Lys His Lys Pro Gly Val

225                 230                 235                 240

Phe Thr Ser Val Cys Tyr Leu Phe Glu Lys Tyr Asn Met Glu Val Leu

                245                 250                 255

Phe Ala Asn Val Ser Ser Asn Val Phe Trp Ser Thr Tyr Val Ile Gln

            260                 265                 270

Ala Gln Val Asn Pro Ser Cys Glu Asn Gln Leu Leu Gly Asn Gly Leu

        275                 280                 285

Gly Val Val Asp Val Phe Lys Gln Val Ser Gln Glu Leu Val Leu Tyr

    290                 295                 300

Phe Ser Ser Leu

305

<210> 9

<211> 53

<212> DNA

<213> Artificial Sequence

<220>

<223> Description of Artificial Sequence: synthetic DNA

<400> 9
ggggacaagt ttgtacaaaa aagcaggctc gatgcccatg ttaatggcga cac          53

<210> 10
<211> 59
<212> DNA
<213> Artificial Sequence

<220>
<223> Description of Artificial Sequence: synthetic DNA

<400> 10
ggggaccact ttgtacaaga aagctgggtt tagcaatcaa ggaaatgatt tgaaaagcc   59

<210> 11
<211> 53
<212> DNA
<213> Artificial Sequence

<220>
<223> Description of Artificial Sequence: synthetic DNA

<400> 11
ggggacaagt ttgtacaaaa aagcaggctc gatggtggcg aaactgtcta tcg          53

<210> 12
<211> 52
<212> DNA

<213> Artificial Sequence

<220>
<223> Description of Artificial Sequence: synthetic DNA

<400> 12
ggggaccact ttgtacaaga aagctgggtt taccgttcta atccagtgac cg          52

<210> 13
<211> 59
<212> DNA
<213> Artificial Sequence

<220>
<223> Description of Artificial Sequence: synthetic DNA

<400> 13
ggggacaagt ttgtacaaaa aagcaggctc gatgagtaat cccgagaagt ctaaaattg  59

<210> 14
<211> 55
<212> DNA
<213> Artificial Sequence

<220>
<223> Description of Artificial Sequence: synthetic DNA

<400> 14
ggggaccact ttgtacaaga aagctgggtt cactcgactt tatcatcgtt ctctt      55

<210> 15

<211> 53

<212> DNA

<213> Artificial Sequence

<220>

<223> Description of Artificial Sequence: synthetic DNA

<400> 15

ggggacaagt ttgtacaaaa aagcaggcta tgactaatgc tcaagagttg ggg          53

<210> 16

<211> 54

<212> DNA

<213> Artificial Sequence

<220>

<223> Description of Artificial Sequence: synthetic DNA

<400> 16

ggggaccact ttgtacaaga aagctgggtt taaagtgaaa agtccaagct aatc          54